# EUROPEAN PATENT SPECIFICATION

(11) **EP 4 305 209 B1**
(45) Date of publication and mention of the grant of the patent: **29.10.2025**
(21) Application number: 22713083.8
(22) Date of filing: 08.03.2022
(51) Int. Cl.: C12Q 1/6886

(54) **MICRORNAS AS BIOMAKERS FOR THE IN VITRO DIAGNOSIS OF GLIOMA**
MIKRO-RNAS ALS BIOMARKER ZUR IN-VITRO-DIAGNOSE VON GLIOM
IN VITRO MIARN UTILISÉS COMME BIOMARQUEURS POUR LE DIAGNOSTICDU GLIOME

(30) Priority: 08.03.2021 IT 202100005357
(43) Date of publication of application: 17.01.2024
(73) Proprietor: Istituti Fisioterapici Ospitalieri, 00144 Roma (IT)
(72) Inventor: RIZZO, Maria, 00144 Roma (RM) (IT); REGAZZO, Giulia, 00144 Roma (RM) (IT); DÍAZ MÉNDEZ, Ana Belén, 00144 Roma (RM) (IT); SACCONI, Andrea, 00144 Roma (RM) (IT)
(74) Representative: Gitto, Serena
(86) International application number: PCT/IT2022/050046
(87) International publication number: WO 2022/190151

(56) References cited:
- WO-A1-2016/186987
- US-A1- 2019 032 054
- US-A1- 2020 040 338
- ANONYMOUS: "TaqMan Array MicroRNA Cards, TaqMan OpenArray MicroRNA Plates and Megaplex(TM) Primer Pools: Target List File (TLF); Version 20.1", INTERNET CITATION, 18 March 2014 (2014-03-18), pages 1 - 62, XP008185879, Retrieved from the Internet <URL:https://www.thermofisher.com/order/catalog/product/4398967>
- GULLUOGLU SUKRU ET AL: "Simultaneous miRNA and mRNA transcriptome profiling of glioblastoma samples reveals a novel set of OncomiR candidates and their target genes", BRAIN RESEARCH, ELSEVIER, AMSTERDAM, NL, vol. 1700, 31 August 2018 (2018-08-31), pages 199 - 210, XP085528713, ISSN: 0006-8993, DOI: 10.1016/J.BRAINRES.2018.08.035
- KIT O I ET AL: "The Role of MicroRNA in Regulation of Signaling Pathways in Gliomas", BIOCHEMISTRY (MOSCOW). SUPPLEMENT SERIES B: BIOMEDICAL CHEMISTRY, MAIK NAUKA - INTERPERIODICA, RU, vol. 12, no. 1, 2 March 2018 (2018-03-02), pages 1 - 21, XP036446714, ISSN: 1990-7508, [retrieved on 20180302], DOI: 10.1134/S1990750818010079

## Description

The present invention concerns microRNAs as biomakers for the *in vitro* diagnosis of glioma. In particular, the present invention concerns microRNAs as biomakers for the *in vitro* diagnosis of glioma and/or for the *in vitro* discrimination of a glioma with a mutation in the IDH1 gene from a glioma with IDH1 wild-type gene.

It is known that gliomas, the most common intrinsic brain tumours arising from progenitor cells in the central nervous system, represent a medical challenge due to their anatomical location, diffuse, infiltrative growth, the resulting impact on brain functioning and their biological complexity [1].

Gliomas encompass a range of different molecular subtypes, many of which have a well-defined profile of driver mutations [2-5]. This biomarker-driven classification is reflected in the 2016 update of the World Health Organization (WHO) classification of central nervous system tumours. This has resulted in the concept of an integrated diagnosis, composed of a histological diagnosis and a molecular profile [6-9].

Among the key genetic events, the isocitrate dehydrogenase 1 (IDH1) mutation is noteworthy from both a diagnostic and a prognostic point of view [3,10]. Alteration of IDH1 gene is a well-established driver mutation in gliomas, identified by the WHO brain tumour classification guidelines as one of the main molecular markers for glioma patients' stratification. In particular, mutations in IDH1 gene are observed at high frequencies in WHO grade II and III astrocytomas, oligodendrogliomas, oligoastrocytomas and in glioblastomas originating from such lower grade neoplasms (secondary glioblastomas) [11]. Moreover, the clinical outcome for tumours with identical histology was different for IDH-wild-type (IDH1wt) and IDH-mutant (IDH1mut) diffuse gliomas, with poorer outcomes in patients with wild-type genotype [11]. The evaluation of IDH1 mutations is commonly performed by molecular profiling of tumour biopsies, however, this could represent a risk for patients not amenable to surgery.

In fact, even if a majority of gliomas will require surgical intervention to relieve the intracranial pressure and reduce tumour burden, a proportion of tumours are located in neurologically sensitive areas and a biopsy poses a significant risk of a deficit [12].

In addition, a most of gliomas inevitably recurs and monitoring the tumour burden of the recurrence is currently achieved by imaging. However, most imaging modalities have limitations in assessing tumour burden, tumour infiltration into adjacent brain, and sometimes imaging is unable to discriminate between tumour recurrence and pseudo-progression [12].

The limited availability of tumour material has made it demanding the identification of alternative and more accessible sources of biomarkers in order to integrate the molecular analyses routinely performed on tissue biopsies. Therefore, the lack of non-invasive biomarkers to monitor growth, progression and response to therapies of these tumours must be considered a major problem in clinical practice. For this reason, clinical studies about innovative molecular procedures and techniques are particularly demanded. To overcome this problem, liquid biopsies, in contrast to tissues biopsies, have the advantage of being minimally invasive, allowing multiple sampling for disease monitoring, and potentially provide, coupled with advanced molecular technologies, the possibility of assessing a wide landscape of molecular markers that the tumour sheds in body fluids. This allows a dynamic patient's management and a personalized medicine approach.

In this context, microRNAs (miRNAs), small non-coding RNA negatively regulating gene expression, are involved in many biological processes and their expression is altered in several diseases, including gliomas [13,14]. miRNAs are deregulated not only in tumour tissues but also in biofluids, such as serum and plasma, [15-17] where they circulate in a very stable form. Studies on circulating miRNAs have evidenced that they can be released in a tissue-specific way [19]. All these characteristics make these small molecules interesting candidates as non-invasive biomarkers [18].

In glioma patients, miRNAs expression is frequently altered in biofluids such blood or cerebrospinal fluid, showing great potential as glioma marker with a range of sensibility of ~30-99 % and specificity around 70-100 % [20]. Some studies have also observed a correlation between miRNA profiles and patients' clinical outcome suggesting a role as prognostic or predictive biomarkers [20].

However, up to date, miRNAs are not yet used in clinical practice for gliomas. This may be related to some limitations concerning the studies about this topic. Indeed, despite the elevated number of circulating RNA species investigated until now, only few reports show consistent results. This lack of reproducibility could be due to a poor uniformity in the researches performed in this field, mainly regarding the study design, the sample size, the ethnicity of the investigated subjects, and the applied methodologies [20,21]. Another limitation is related to the lack of studies about correlations between miRNA profiles and molecular features of gliomas, despite the importance the molecular markers have gained after the updated WHO classification of brain tumours in 2016 [22-25].

Across all the published studies, several circulating miRNAs have been evaluated in gliomas as single biomarkers [16,17,20,23-30]. However, it is widely recognized that single miRNA profiles may provide a low accuracy as cancer biomarkers, mostly due to the multifactorial nature of tumour and to the large number of targets for a single miRNA [15-17,20,23-30]. Thus, the evaluation of a multiple miRNA signature could be a more valuable and reliable approach to identify molecular markers that could mirror the complexity of the disease.

According to GULLUOGLU SUKRU ET AL, BRAIN RESEARCH, ELSEVIER, AMSTERDAM, NL, vol. 1700, 31 August 2018, pages 199-210, simultaneous miRNA and mRNA transcriptome profiling of glioblastoma samples reveals a novel set of OncomiR candidates and their target genes.

KIT O I ET AL, BIOCHEMISTRY (MOSCOW), SUPPLEMENT SERIES B: BIOMEDICAL CHEMISTRY, MAIK NAUKA - INTERPERIODICA, RU, vol. 12, no. 1, 2 March 2018, pages 1-21, relates to the Role of MicroRNA in Regulation of Signaling Pathways in Gliomas.

In the light of the above, it is therefore apparent the need to provide new tools for the diagnosis and monitoring of glioma patients, able to overcome the disadvantages of known methods.

According to the present invention it has now been found that glioma can be diagnosed *in vitro* by measuring the expression levels of combined serum miRNAs miR-1-3p, miR-26a-1-3p and miR-487b-3p in comparison to the expression levels thereof in healthy subjects. In addition, the above-mentioned miRNAs can be advantageously used in order to discriminate with a high sensibility and sensitivity glioma patients with a mutation in the IDH1 gene from subjects with the wild-type genotype. Moreover, these identified miRNA signature have been shown to have a prognostic value in glioma patients since they are correlated with progression-free survival (PFS).

The miRNAs detection according to the present invention are obtainable by minimally invasive blood sampling, and they can represent a useful source of information complementary to the current histopathological, molecular and imaging analysis, in order to improve patients' diagnosis, prognosis and monitoring.

The method according to the present invention advantageously offers a significant contribution to patients' managements since it is rapid, cheap and it could: a) improve patients' stratification giving information about the molecular features of the tumour also in patients non amenable to tissue biopsy; b) allow a non-invasive monitoring of the disease over the time, also during relapse or follow-up.

Therefore, according to the present invention, a non-invasive, rapid and cheap blood test can be used for the detection of the above-mentioned biomarkers for diagnosis and monitoring of glioma patients. The use of the present invention in clinical settings can significantly contribute to improve glioma management, with critical impact on the efficacy and economic burden of service offered by the National Health System

Moreover, these findings have significant potential to transform current diagnostic paradigms, as well as to provide alternative endpoints for future clinical trials and thus can be considered a pathway towards personalized medicine.

It is therefore specific object of the present invention a method for the *in vitro* diagnosis of gliomas and/or for the *in vitro* discrimination of a glioma with a mutation in the IDH1 gene from a glioma with IDH1 wild-type gene, said method comprising or consisting of measuring (or obtaining a measurement of) the expression, in a biological sample, of all the three miRNAs miR-1-3p, miR-26a-1-3p and miR-487b-3p,
wherein said miRNAs are under-expressed in a biological sample of a glioma patient in comparison to the expression thereof in a biological sample of a healthy subject and said miRNAs are over-expressed in a biological sample of a IDH1-mutated glioma patient in comparison to the expression thereof in a biological sample of an IDH1-wild type glioma patient.

The gliomas that can be diagnosed according to the method of the present invention can be divided in four histological grades and comprehend several subtypes such as astrocytomas, oligodendrogliomas, ependymomas, primary or secondary glioblastomas.

According to the method of the present invention, said biological sample can be a liquid biopsy, such as blood sample, serum sample, plasma sample, saliva sample, urine sample, cerebrospinal fluid sample.

According to the method of the present invention, therefore, the expression of said microRNAs can be measured by using pairs of primers and/or probes suitable for the purpose. It is known that primers and probes are oligonucleotide sequences complementary to the microRNA sequences to be detected. According to the present invention, said method can be carried out by using one or more synthetic sequences complementary to at least a part of miR-1-3p, one or more synthetic sequences complementary to at least a part of miR-26a-1-3p and one or more synthetic sequences complementary to at least a part of miR-487b-3p. In particular, said one or more synthetic sequences can be primers and/or probes.

According to the present invention, said method can be carried out by small RNA Next Generation Sequencing, Microarray, Nanostring based methods, Real Time PCR, Digital or Droplet Digital PCR, RNA Hybridization Methods such as Northern Blot or Dot Blot.

The expression levels of the three selected miRNAs can be evaluated, for example by digital or droplet digital PCR, in biological samples, such as serum samples, obtained from patients before and/or after surgery and processed following a standardized protocol to reduce pre-analytical variables. Therefore, the evaluation of the expression of the three miRNAs of the invention can be advantageously used for the patient's follow up. In addition, the expression levels of the three selected miRNAs can be evaluated when a surgery is not feasible or risky for the patient.

Disclosed, but not part of the claimed invention, is a combination of miRNAs as biomarkers for the *in vitro* diagnosis of gliomas and/or for the *in vitro* discrimination of a glioma with a mutation in the IDH1 gene from a glioma with IDH1 wild-type gene, wherein said combination of miRNAs comprises or consists of miR-1-3p, miR-26a-1-3p and miR-487b-3p. As mentioned above, said miRNAs miR-1-3p, miR-26a-1-3p and miR-487b-3p are under-expressed in a biological sample of a glioma patient in comparison to the expression thereof in a biological sample of a healthy subject and are over-expressed in a biological sample of a IDH1-mutated glioma patient in comparison to the expression thereof in a biological sample of a IDH1-wild type glioma patient.

The present invention concerns also the use of a combination as defined above as biomarker for the *in vitro* diagnosis of gliomas and/or for the *in vitro* discrimination of a glioma with a mutation in the IDH1 gene from a glioma with IDH1 wild-type gene. As mentioned above, said miRNAs miR-1-3p, miR-26a-1-3p and miR-487b-3p are under-expressed in a biological sample of a glioma patient in comparison to the expression thereof in a biological sample of a healthy subject and are over-expressed in a biological sample of a IDH1-mutated glioma patient in comparison to the expression thereof in a biological sample of an IDH1-wild type glioma patient.

The present invention concerns also a kit for the *in vitro* diagnosis of gliomas and/or for the *in vitro* discrimination of a glioma with a mutation in the IDH1 gene from a glioma with IDH1 wild-type gene, said kit comprising or consisting of three primer pairs and/or three probes for the detection of all the three miRNAs miR-1-3p, miR-26a-1-3p and miR-487b-3p. According to the present invention, the kit does not comprise any other primer pair and/or probe for the detection of other miRNAs, i.e., other natural miRNAs, whereas the kit can comprise primers and/or probes for detecting synthetic oligonucleotides for example spike-ins used to normalize the hybridization measurements.

Therefore, the kit according to the present invention can be used in a method for the *in vitro* diagnosis of gliomas and/or for the *in vitro* discrimination of a glioma with a mutation in the IDH1 gene from a glioma with IDH1 wild-type gene.

The kit according to the present invention can further comprise other reagents suitable for measuring the expression levels of said three miRNAs, such as one or more enzymes.

Disclosed, but not part of the claimed invention, is a method of treatment of glioma in a subject, said method comprising
obtaining a measurement of the expression, in a biological sample of the subject, all the three miRNAs miR-1-3p, miR-26a-1-3p and miR-487b-3p and,
when said miRNAs are under-expressed in comparison to the expression thereof in a biological sample of a healthy subject and said miRNAs are not over-expressed in comparison to the expression thereof in a biological sample of an IDH1-wild type glioma patient,
treating the subject with radio and/or chemotherapy after surgery, since the subject suffers from IDH1-wild type glioma; whereas
when said miRNAs are under-expressed in comparison to the expression thereof in a biological sample of a healthy subject and said miRNAs are over-expressed in comparison to the expression thereof in a biological sample of an IDH1-wild type glioma patient,
avoiding treating the subject with radio and or chemotherapy after surgery, since the subject suffers from IDH1-mutated type glioma and, if possible, directing the subject to clinical trials with targeted therapies. In particular, this therapy is applied for low grade glioma patients.

The present invention now will be described by an illustrative, but not limitative way, according to preferred embodiments thereof, with particular reference to the examples and the enclosed drawings, wherein:
- **Figure 1** shows a schematic representation of the experimental plan for the global miRNA profiling by small RNA-Seq;
- **Figure 2** shows Kaplan-Meier curves for progression free survival (PFS) based on miRNAs expression levels: **A)** Correlation between the expression of 8 out of 10 miRNAs downregulated in the IDH1wt group and PFS; **B)** Correlation between the expression of 2 out of 10 miRNAs upregulated in the IDH1wt group and PFS; Kaplan-Meier plots: patients were stratified into high and low groups based on miRNA expression z-score (threshold±0);
- **Figure 3** shows the Roc curves, which indicate the diagnostic accuracy of the 10 identified miRNAs, the values of the Area under the curve (AUC) are reported;
- **Figure 4** shows combined ROC curve for miR-1-3p/ miR-26a-1-3p/ miR-487b-3p /signature relative to the discrimination between IDH1wt and mutant patients; area under the curve (AUC), Sensitivity and Specificity values are reported;
- **Figure 5** shows Kaplan-Meier curves for the selected three miRNA signature: correlation between the expression of miR-1-3p/ miR-26a-1-3p/ miR-487b-3p signature and **(A)** overall survival (OS) or **(B)** progression free survival (PFS) of glioma patients; hazard ratio (HR) and confidence interval are reported;
- **Figure 6** shows expression levels of the serum miRNA signature in healthy controls and glioma patients: scatter plots of the expression levels of the indicated miRNAs in serum samples of heathy subjects (controls; n=15) and glioma patients (gliomas; n=15) analysed by ddPCR. P values (*** = P<0.001, ** =P<0.01, * =P<0.05) were calculated by Mann-Whitney test;
- **Figure 7** shows ROC curves, which indicate the diagnostic accuracy of the 3 selected serum miRNAs **(A)** and their combination **(B)** in discriminating glioma patients from healthy subjects; AUC values are reported;
- **Figure 8** shows expression levels of the serum miRNA signature in IDH1 wild-type and IDH1 mutant glioma patients: the Histograms represent the mean expression levels, analysed by ddPCR, of serum miR-1-3p, -26a-1-3p and -487b-3p in serum of IDH1 wild-type (IDH1wt; n=9) and IDH1 mutant (IDH1mut; n=6) glioma patients; bars represent the standard error; P values (* = P<0,05) were calculated by Mann-Whitney test.

### EXAMPLE 1: Identification and study of circulating miRNAs as non-invasive tools for diagnosis and monitoring of glioma patients

### Materials and Methods

### Sample processing

This study was conducted on a cohort of 37 glioma patients with ranging from grade II to IV, recruited at the Regina Elena National Cancer Institute (IRE), which included, matched by gender and age, 27 patients with IDH1-wild-type and 10 patients IDH1 mutant. Sampling method was consistent throughout the study to minimize any pre-analytical variables.

Blood samples of glioma patients were collected at diagnosis in BD Vacutainer serum tubes using a 21-gauge needle. The samples were kept at room temperature (RT) for 30 min and then centrifuged at RT for 20 min at 1100×g, the supernatant was further centrifuged for 5 min at 1300×g. The serum transferred into sterile cryovials was aliquoted and stored at -80 °C until further analysis.

*The study was approved by the Institutional Ethical Committee of IRE. All patients signed an informed consent before inclusion and were treated according to ethical and legal standards adopted by the Declaration of Helsinki.*

### RNA extraction, Reverse Transcription and quantitive Real-Time PCR (qRT-PCR)

Total RNA was extracted from 200µl of serum and using the miRNeasy Serum/Plasma Advanced Kit (Qiagen), in accordance with manufacturer's instructions. RNA spike-in mix (QlAseq miRNA Kit, Qiagen) was added during extraction for successive data normalization.

Complementary DNA (cDNA) was synthetized from 2 µl of the RNA solution extracted from the serum using QIAseq^{™} miRNA Library QC PCR kit II (Qiagen) and following the manufacturer's instructions.

The suitability of the samples for small RNA-sequencing was assessed by qRT-PCR using the miRCURY LNA SYBR Green PCR Kit (Qiagen). In order to evaluate the uniformity of extracted RNA samples, two synthetic RNA spike-ins, UniSp 100 and UniSp 101, were analysed. The typical Cq value is in the range of 31-34 for UniSp 100 and 25-28 for UniSp 101. The ΔCq between these two Spike- ins should be around 5-7. Moreover, haemolysis was assessed evaluating the levels of miR-451, a particularly abundant miRNA in erythrocytes and compared with the amount of miR-23a, a miRNA not altered by haemolysis, following Qiagen protocols (OIAseq miRNA Kit, Qiagen). Haemolysed samples were excluded from further analysis [31].

### Library preparation and small RNA-Sequencing

For miRNA library preparation a starting amount of 5µl of RNA extracted from serum has been processed using the QlAseq miRNA Library Kit (Qiagen) as indicated by the manufacture's protocol. First, adaptors were sequentially ligated to the 3' and 5' ends of miRNAs. Adaptors include molecular bar codes called UMIs (Unique Molecular Indices), enabling unbiased and accurate miRNome-wide quantification of mature miRNAs by NGS. Subsequently, universal cDNA synthesis with UMI assignment, cDNA clean-up, library amplification and library clean-up are performed. Proprietary methodology using modified oligonucleotides virtually eliminates the presence of adapter dimers in the sequencing library, effectively removing a major contaminant often observed during sequencing. Additionally, the kit is designed to also minimize the presence of hY4 Y RNA, which is often observed in high levels in serum and plasma samples.

Yield and size distribution of resultant libraries were validated using Agilent 2100 Bioanalyzer on a High-sensitivity DNA Assay (Agilent Technologies). Libraries were then pooled with an equal proportion for multiplexed sequencing on the Illumina NextSeq550 (Illumina, United States) platform.

### Droplet digital PCR

Absolute quantification of the signature miRNAs expression in serum samples newly recruited glioma patients and age and sex matched heathy subjects has been performed by the QX200 Droplet Digital PCR System (Biorad) using EvaGreen Master Mix (Biorad) and miRNA-specific miRCURY LNA miRNA PCR Assays (Qiagen).

### Bioinformatics and statistical analysis

Data pre-processing was performed using QIAseq miRNA Primary Data Analysis pipeline, followed by genome alignment to human genome using Bowtie 2 tool. UMI counts were normalized by considering a size factor for each sample. To estimate the size factors, we considered the median of the ratios of observed counts to those of a pseudo-reference sample, whose counts can be obtained by considering the geometric mean of each gene across all samples [32]. Then, to transform the observed counts to a common scale, the observed counts in each sample were divided by the corresponding size factor.

Differential expression analysis was performed using a Negative Binomial Model. Linkage between the variance and the mean was established by a locally regressed non-parametric smooth function of the mean. The Benjamini-Hochberg (BH) procedure for multiple testing was used to obtain adjusted P-values.

The Receiver Operative Characteristics (ROC) curves were used to determine the diagnostic accuracy of miRNAs in distinguishing glioma patients with IDH1 wild type from mutant patients (area under the ROC curve measures were ≥0.75). The diagram is a plot of the sensitivity (true-positive rate) vs specificity (false-positive rate) over all possible ΔCT values. Average expression of standardized counts of the miRNA signature was used to fit a binomial model. Prediction scores from the classifier were then considered to evaluate the true positive rate (sensitivity) and the false positive rate (1-specificity) in the ROC curve. Performance of the curves was assessed by calculating the Area Under Curve (AUC) with 1000 bootstrap replicas for computation of the confidence bounds. The Kaplan-Meier curves were used to estimate the overall survival (OS) and progression free survival (PFS) based on miRNAs expression level. Patients were stratified into high and low groups based on miRNA expression z-score.

The required sample size for the validation cohort was estimated on the basis of our preliminary data. Specifically, we based our estimates on the comparison between the mean values (±SD) of the most deregulated miRNA in the three-miRNAs signature, in IDH-wt and IDH-mut glioma cases (2.63 (±1.35) and 3.89 (±0.64), respectively). A Student's T test with an overall sample size of 46 patients (23 for each group) achieved a 90% statistical power and a 0.01 significance level. This estimate includes a 10% of drop-out rate.

As far as the comparison between glioma cases and healthy subjects is concerned a Student's T test with an overall sample size of 42 patients (21 for each group) achieved a 90% statistical power and a 0.05 significance level. Overall a total of 67 subjects, 46 gliomas (23 IDH-wt and 23 IDH-mut) and 21 healthy controls will be collected.

Mann-Whitney test was performed to evaluate the significance of differences in miRNAs expression between groups of subjects.

### Results

### Global expression profiling of circulating miRNAs:

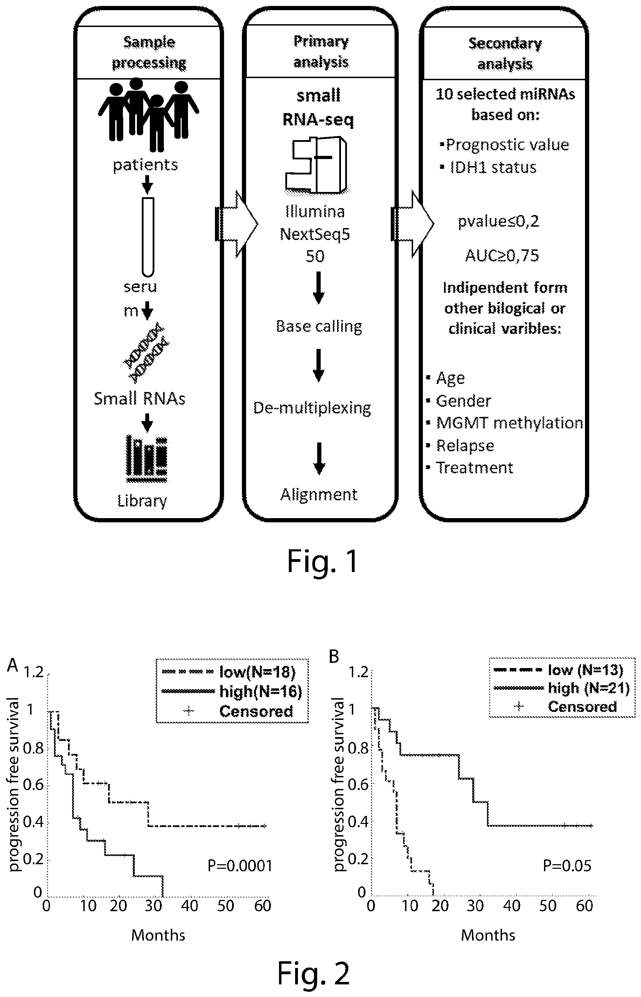

To assess if circulating miRNAs could mirror the mutational status of IDH1 the serum-miRNome of glioma patients was analysed. To this purpose individual serum samples from a training cohort of glioma patients with different IDH1 mutational status (n=27 IDH1wt and n=10 IDH1mut) were analysed by small RNA-Sequencing (Figure 1). Based on these results, ten miRNAs were subsequently selected with prognostic value and dysregulated between IDH1wt and IDH1mut patients (two upregulated and eight downregulated in the group of IDH1wt patients *versus* mutants; Table 1).

Table 1 shows miRNAs selected by small RNA-Seq.

**Table 1**

| **miRNA** | **MIMAT code** | **IDH1wt *vs* IDH1mut** | **Fold change** | **Area under the ROC curve** |
|---|---|---|---|---|
| hsa-miR-1-3p | MIMAT0000416 | Under-expressed | 1,6 | 0,866 |
| hsa-miR-26a-1-3p | MIMAT0004499 | Under-expressed | 1,9 | 0,825 |
| hsa-miR-127-3p | MIMAT0000446 | Under-expressed | 1,7 | 0,754 |
| hsa-miR-130b-5p | MIMAT0004680 | Under-expressed | 1,3 | 0,866 |
| hsa-miR-485-3p | MIMAT0002176 | Under-expressed | 1,6 | 0,766 |
| hsa-miR-487b-3p | MIMAT0003180 | Under-expressed | 1,6 | 0,754 |
| hsa-miR-493-5p | MIMAT0002813 | Under-expressed | 1,7 | 0,771 |
| hsa-miR-542-3p | MIMAT0003389 | Over-expressed | 1,4 | 0,76 |
| hsa-miR-589-5p | MIMAT0004799 | Under-expressed | 1,3 | 0,836 |
| hsa-miR-4778-5p | MIMAT0019936 | Over-expressed | 1,7 | 0,754 |

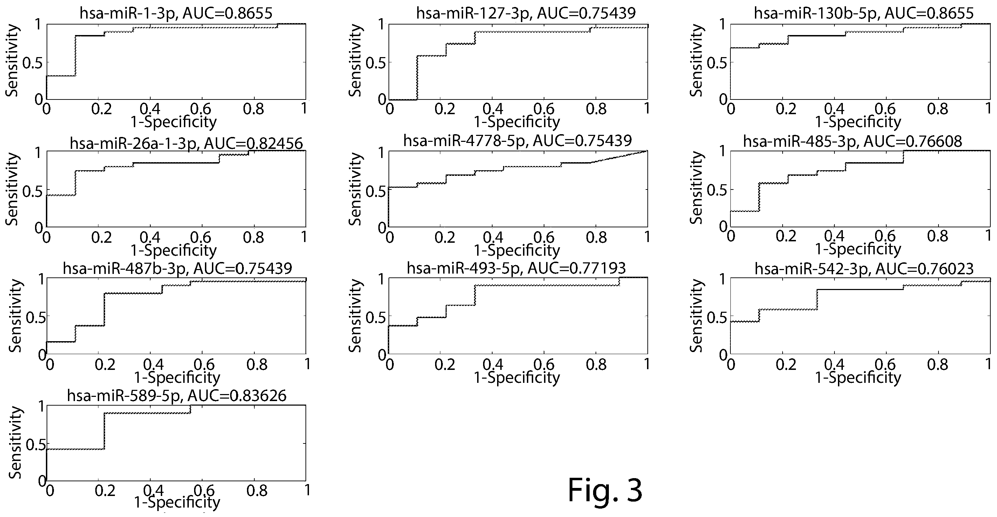

The prognostic impact was evaluated by Kaplan Meyer curves showing that the expression of these miRNAs is significantly and independently correlated with patients' Progression-Free Survival (PFS), both considering the miRNAs downregulated in IDH1wt (Figure 2A; hazard ratio:0.24, 95% CI: 0.12-0.47) and the upregulated ones (Figure 2B; hazard ratio:2.1, 95% CI: 1.16-3.83). ROC curve analyses were performed to assess the discrimination power based on IDH1 status. The 10 selected miRNAs showed an area under the curve (AUC) value ranging from 0.75 to 0.86, indicating the high diagnostic accuracy of all the identified miRNAs (Figure 3).

### Combined miRNA analysis:

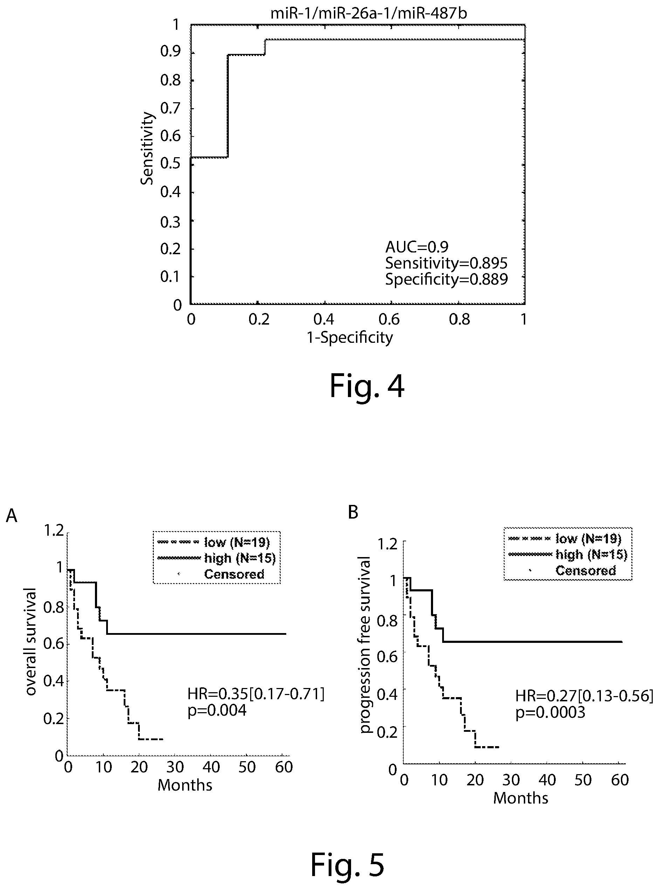

Different combinations of the selected miRNAs have been subsequently considered to evaluate if they could provide an improvement of the diagnostic accuracy. As shown in Figure 4A and B, ROC curves analyses indicate that the combination of miR-1-3p/ miR-26a-1-3p/ miR-487b-3p led to a substantial improvement in the diagnostic performance (AUC 0.9), compared to the single miRNAs of the signature.

Kaplan-Meier analysis was performed to assess the prognostic value of this three-miRNA signature. As shown in Figure 5 the combined miRNA signature was significantly correlated to both overall Survival (OS) and PFS of glioma patients.

Altogether these findings indicate a considerable importance of this restricted serum-miRNA signature as highly specific and sensitive non-invasive diagnostic/prognostic biomarker for a personalized medicine approach in glioma patients.

### The miR-1-3p/miR-26a-1-3p/miR-487b-3p signature discriminates glioma patients from healthy subjects

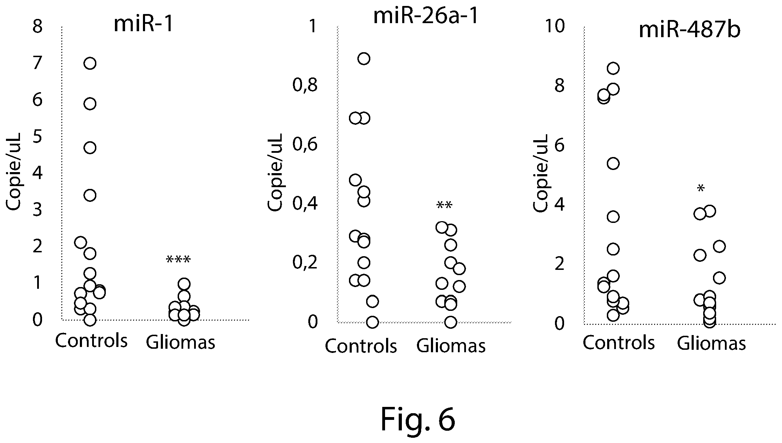

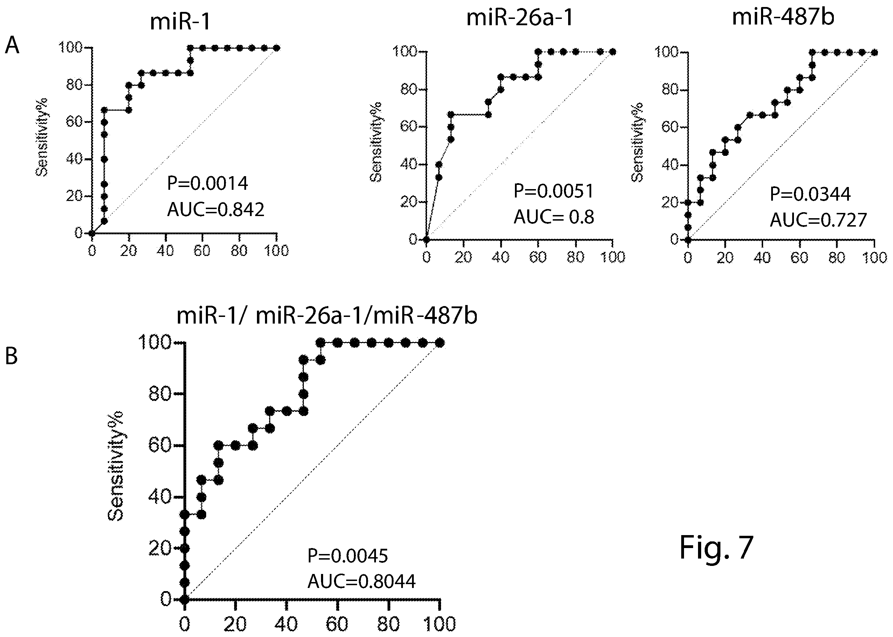

To evaluate the specificity of the selected miRNAs for the pathology of interest (gliomas), their expression was evaluated, by droplet-digital PCR (ddPCR), in serum samples of a new cohort of glioma patients (n=15, of which 9 IDH1wt and 6 IDH1mut) and of sex and age paired healthy subjects (n=15). The results show that all the members of this restricted signature are significantly downregulated in serum of glioma patients compare to healthy controls (Figure 6) and can discriminate with a good accuracy these two groups of subjects (Figure 7).

### Validation of the restricted miRNA signature:

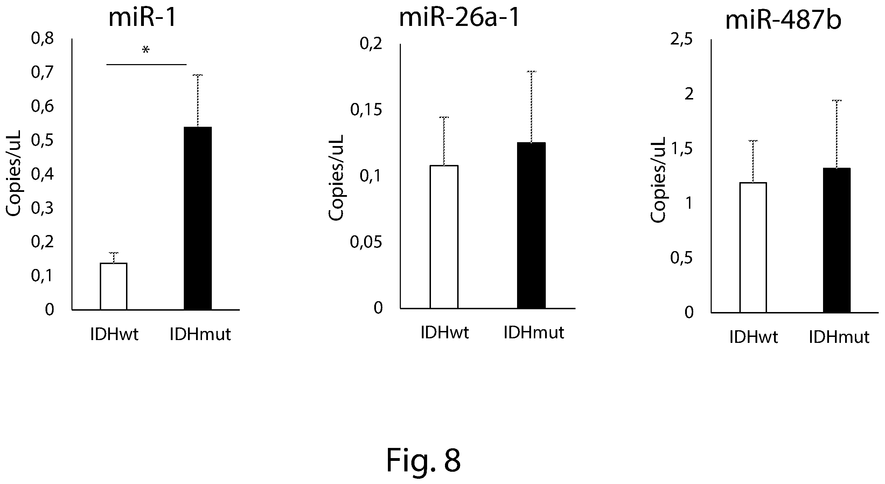

As part of a multicentre study entitled "Validation study of a serum miRNA signature correlated to IDH1 mutation status as non-invasive diagnostic and prognostic biomarker in glioma patients", performed in collaboration with "Carlo Besta" Neurological Institute, the enrolment of a new and wider cohort of glioma patients (IDHwt n=22, IDHmut n=22) is ongoing with the aim of validating the results obtained in the training set. Preliminary data, on the first 15 recruited patients, confirm the results obtained in genome-wide profiling (Figure 8). To further corroborate these data, assessment of the signature expression levels by *in vitro* experiments are currently underway to recapitulate what observed in patients using glioma cell lines engineered with different mutational status of the IDH1 gene.

### References

1. McFaline-Figueroa JR, Lee EQ. Brain Tumors. Am J Med. 2018;131(8):874-882
2. Molinari E, Curran OE, Grant R. Clinical importance of molecular markers of adult diffuse glioma. Pract Neurol. 2019; 19: 412-416.
3. Capper D, Weissert S, Balss J, Habel A, Meyer J, Jäger D et al. Characterization of R132H mutation-specific IDH1 antibody binding in brain tumors. Brain Pathol. 2010; 20: 245-254.
4. Capper D, Preusser M, Habel A, Sahm F, Ackermann U, Schindler G, et al. Assessment of BRAF V600E mutation status by immunohistochemistry with a mutation-specific monoclonal antibody. Acta Neuropathol. 2011; 122: 11-19.
5. Khuong-Quang DA, Buczkowicz P, Rakopoulos P, Liu XY, Fontebasso AM, Bouffet E et al. K27M mutation in histone H3.3 defines clinically and biologically distinct subgroups of pediatric diffuse intrinsic pontine gliomas. Acta Neuropathol. 2012; 124: 439-447.
6. Louis DN, Wesseling P, Aldape K, Brat DJ, Capper D, Cree IA et al. cIMPACT-NOW update 6: new entity and diagnostic principle recommendations of the cIMPACT-Utrecht meeting on future CNS tumor classification and grading. Brain Pathol. 2020; 30: 844-856.
7. Brat DJ, Aldape K, Colman H, Figrarella-Branger D, Fuller GN, Giannini C et al. cIMPACT-NOW update 5: recommended grading criteria and terminologies for IDH-mutant astrocytomas. Acta Neuropathol 2020; 139: 603-608.
8. Brat DJ, Aldape K, Colman H, Holland EC, Louis DN, Jenkins RB, et al. cIMPACT-NOW update 3: recommended diagnostic criteria for "Diffuse astrocytic glioma, IDH-wildtype, with molecular features of glioblastoma, WHO grade IV". Acta Neuropathol 2018; 136: 805-810.
9. Capper D, Jones DTW, Sill M, Hovestadt V, Schrimpf D, Sturm D et al. DNA methylation-based classification of central nervous system tumours. Nature. 2018; 555: 469-474.
10. Han S, Liu Y, Cai SJ, Qian M, Ding J, Larion M, Gilbert MR, Yang C. IDH mutation in glioma: molecular mechanisms and potential therapeutic targets. Br J Cancer. 2020 May;122(11):1580-1589.
11. Wesseling P, Capper D. WHO 2016 Classification of gliomas. Neuropathol Appl Neurobiol. 2018;44(2):139-150.
12. Bagley SJ, Nabavizadeh SA, Mays JJ, Till JE, Ware JB, Levy S et al. Clinical Utility of Plasma Cell-Free DNA in Adult Patients with Newly Diagnosed Glioblastoma: A Pilot Prospective Study. Clin Cancer Res. 2020;26:397-407.
13. Chen R, Smith-Cohn M, Cohen AL, Colman H. Glioma Subclassifications and Their Clinical Significance. Neurotherapeutics. 2017 Apr;14(2):284-297.
14. Odjélé A, Charest D, Morin Jr P. miRNAs as important drivers of glioblastomas: a no-brainer? Cancer Biomark. 2012;11:245-52
15. Kosaka N, Iguchi H, Ochiya T. Circulating microRNA in body fluid: a new potential biomarker for cancer diagnosis and prognosis. Cancer Sci. 2010 Oct;101(10):2087-92.
16. Westphal M, Lamszus K. Circulating biomarkers for gliomas. Nat Rev Neurol. 2015 Oct;11(10):556-66.
17. Garcia CM, Toms SA. The Role of Circulating MicroRNA in Glioblastoma Liquid Biopsy. World Neurosurg. 2020 Jun;138:425-435.
18. He Y et al, "Current State of Circulating MicroRNAs as Cancer Biomarkers". Clinical Chemistry 2015, 61:9 1138-1155.
19. Yang C, Wang C, Chen X, Chen S, Zhang Y, Zhi F et al. Identification of seven serum microRNAs from a genome-wide serum microRNA expression profile as potential noninvasive biomarkers for malignant astrocytomas. Int J Cancer. 2013; 132: 116-127.
20. Diaz Méndez AB, Tremante E, Regazzo G, Brandner S, Rizzo MG. Time to focus on circulating nucleic acids for diagnosis and monitoring of gliomas: a systematic review of their role as biomarkers. Neuropathol Appl Neurobiol. 2021 Jan 5. doi: 10.1111/nan.12691. Online ahead of print.
21. Shi R, Wang PY, Li XY, Chen JX, Li Y, Zhang XZ et al. Exosomal levels of miRNA-21 from cerebrospinal fluids associated with poor prognosis and tumor recurrence of glioma patients. Oncotarget. 2015; 6: 26971-26981.
22. Ebrahimkhani S, Vafaee F, Hallal S, Wei H, Lee MYT, Young PE et al. Deep sequencing of circulating exosomal microRNA allows non-invasive glioblastoma diagnosis. NPJ Precis Oncol. 2018; 2: 28-018-0071-0.
23. Zhong F, Huang T, Leng J. Serum miR-29b as a novel biomarker for glioblastoma diagnosis and prognosis. Int J Clin Exp Pathol. 2019; 12: 4106-4112.
24. Zhang H, Wang J, Wang Z, Ruan C, Wang L, Guo H. Serum miR-100 is a potential biomarker for detection and outcome prediction of glioblastoma patients. Cancer Biomark. 2019; 24: 43-49.
25. Zhang Y, Ta WW, Sun PF, Meng YF, Zhao CZ. Diagnostic and prognostic significance of serum miR-145-5p expression in glioblastoma. Int J Clin Exp Pathol. 2019; 12: 2536-2543.
26. Regazzo G, Terrenato I, Spagnuolo M, Carosi M, Cognetti G, Cicchillitti L et al. A restricted signature of serum miRNAs distinguishes glioblastoma from lower grade gliomas. J Exp Clin Cancer Res. 2016; 35: 124-016-0393-0.
27. Wang J, Che F, Zhang J, Zhang M, Xiao S, Liu Y et al. Diagnostic and Prognostic Potential of Serum Cell-Free microRNA-214 in Glioma. World Neurosurg. 2019; 125: e1217-e1225.
28. Parviz Hamidi M, Haddad G, Ostadrahimi S, Ostadrahimi N, Sadeghi S, Fayaz S et al. Circulating miR-26a and miR-21 as biomarkers for glioblastoma multiform. Biotechnol Appl Biochem. 2019; 66: 261-265.
29. Lan F, Yue X, Xia T. Exosomal microRNA-210 is a potentially non-invasive biomarker for the diagnosis and prognosis of glioma. Oncol Lett. 2020; 19: 1967-1974.
30. Wang LQ, Sun W, Wang Y, Li D, Hu AM. Downregulation of plasma miR-124 expression is a predictive biomarker for prognosis of glioma. Eur Rev Med Pharmacol Sci. 2019; 23: 271-276.
31. Blondal T, Jensby Nielsen S, Baker A, Andreasen D, Mouritzen P, Wrang Teilum M et al. Assessing sample and miRNA profile quality in serum and plasma or other biofluids. Methods. 2013;59(1):S1-6.
32. Anders S, Huber W. Differential expression analysis for sequence count data. Genome Biol. 2010;11(10): R106.

## Claims

1. Method for the *in vitro* diagnosis of gliomas and/or for the *in vitro* discrimination of a glioma with a mutation in the IDH1 gene from a glioma with IDH1 wild-type gene, said method comprising or consisting of measuring the expression, in a biological sample, of all the three miRNAs miR-1-3p, miR-26a-1-3p and miR-487b-3p,
wherein said miRNAs are under-expressed in a biological sample of a glioma patient in comparison to the expression thereof in a biological sample of a healthy subject and said miRNAs are over-expressed in a biological sample of a IDH1-mutated glioma patient in comparison to the expression thereof in a biological sample of an IDH1-wild type glioma patient.

2. Method according to claim 1, wherein the biological sample is a liquid biopsy.

3. Method according to claim 2, wherein said liquid biopsy is selected from the group consisting of blood sample, serum sample, plasma sample, saliva sample, urine sample, cerebrospinal fluid sample.

4. Method according to any one of claims 1-3, wherein said method is carried out by using one or more synthetic sequences complementary to at least a part of miR-1-3p, one or more synthetic sequences complementary to at least a part of miR-26a-1-3p and one or more synthetic sequences complementary to at least a part of miR-487b-3p.

5. Method according to claim 4, wherein said one or more synthetic sequences are primers and/or probes.

6. Method according to any one of claims 1-5, wherein said method is carried out by small RNA Next Generation Sequencing, Nanostring based methods, Microarray, Real Time PCR, Digital or Droplet Digital PCR, RNA Hybridization Methods such as Northern Blot or Dot Blot.

7. Use of a combination of miRNAs as biomarker for the *in vitro* diagnosis of gliomas and/or for the *in vitro* discrimination of a glioma with a mutation in the IDH1 gene from a glioma with IDH1 wild-type gene, wherein said combination of miRNAs comprises or consists of all miR-1-3p, miR-26a-1-3p and miR-487b-3p, wherein said miRNAs are under-expressed in a biological sample of a glioma patient in comparison to the expression thereof in a biological sample of a healthy subject and said miRNAs are over-expressed in a biological sample of a IDH1-mutated glioma patient in comparison to the expression thereof in a biological sample of an IDH1-wild type glioma patient.

8. Kit for the *in vitro* diagnosis of gliomas and/or for the *in vitro* discrimination of a glioma with a mutation in the IDH1 gene from a glioma with IDH1 wild-type gene, said kit comprising or consisting of three primer pairs and/or three probes for the detection of all the three miRNAs miR-1-3p, miR-26a-1-3p and miR-487b-3p, with the proviso that the kit does not comprise any other primer pair and/or probe for the detection of other natural miRNAs.

## Patentansprüche

1. Verfahren zur *in vitro*-Diagnose von Gliomen und/oder zur *in vitro*-Unterscheidung eines Glioms mit einer Mutation im IDH1-Gen von einem Gliom mit IDH1-Wildtyp-Gen, wobei das Verfahren die Messung der Expression aller drei miRNA miR-1-3p, miR-26a-1-3p und miR-487b-3p in einer biologischen Probe umfasst oder daraus besteht,
wobei die miRNA in einer biologischen Probe eines Gliompatienten im Vergleich zu ihrer Expression in einer biologischen Probe eines gesunden Subjekts unterexprimiert sind und die miRNA in einer biologischen Probe eines IDH1-mutierten Gliompatienten im Vergleich zu ihrer Expression in einer biologischen Probe eines IDH1-Wildtyp-Gliompatienten überexprimiert sind.

2. Verfahren nach Anspruch 1, wobei die biologische Probe eine Flüssigbiopsie ist.

3. Verfahren nach Anspruch 2, wobei die Flüssigbiopsie ausgewählt ist aus der Gruppe bestehend aus Blut-, Serum-, Plasma-, Speichel-, Urin- und Liquorproben.

4. Verfahren nach einem der Ansprüche 1 bis 3, wobei das Verfahren unter Verwendung einer oder mehrerer synthetischer Sequenzen, die zu mindestens einem Teil von miR-1-3p komplementär sind, einer oder mehrerer synthetischer Sequenzen, die zu mindestens einem Teil von miR-26a-1-3p komplementär sind, und einer oder mehrerer synthetischer Sequenzen, die zu mindestens einem Teil von miR-487b-3p komplementär sind, durchgeführt wird.

5. Verfahren nach Anspruch 4, wobei die eine oder mehreren synthetischen Sequenzen Primer und/oder Sonden sind.

6. Verfahren nach einem der Ansprüche 1-5, **dadurch gekennzeichnet, dass** das Verfahren mittels Small-RNA-Next-Generation-Sequenzierung, Nanostring-basierten Verfahren, Microarray, Real-Time-PCR, Digital- oder Droplet-Digital-PCR oder RNA-Hybridisierungsverfahren wie Northern Blot oder Dot Blot durchgeführt wird.

7. Verwendung einer Kombination von miRNA als Biomarker für die *in vitro* Diagnose von Gliomen und/oder für die *in vitro* Unterscheidung eines Glioms mit einer Mutation im IDH1-Gen von einem Gliom mit IDH1-Wildtyp-Gen, wobei die Kombination von miRNA alle miR-1-3p, miR-26a-1-3p und miR-487b-3p umfasst oder daraus besteht, wobei die miRNA in einer biologischen Probe eines Gliompatienten im Vergleich zu ihrer Expression in einer biologischen Probe eines gesunden Subjekts unter- exprimiert sind und die miRNA in einer biologischen Probe eines IDH1-mutierten Gliompatienten im Vergleich zu ihrer Expression in einer biologischen Probe eines IDH1-Wildtyp-Gliompatienten überexprimiert sind.

8. Kit für die *in vitro* Diagnose von Gliomen und/oder für die *in vitro* Unterscheidung eines Glioms mit einer Mutation im IDH1-Gen von einem Gliom mit IDH1-Wildtyp-Gen, wobei der Kit drei Primerpaare und/oder drei Sonden für den Nachweis aller drei miRNA miR-1-3p, miR-26a-1-3p und miR-487b-3p umfasst oder daraus besteht, mit der Maßgabe, dass der Kit kein anderes Primerpaar und/oder keine andere Sonde für den Nachweis anderer natürlicher miRNA umfasst.

## Revendications

1. Méthode pour le diagnostic *in vitro* des gliomes et/ou pour la discrimination *in vitro* d'un gliome avec une mutation dans le gène IDH1 d'un gliome avec le gène IDH1 de type sauvage, ladite méthode comprenant ou consistant à mesurer l'expression, dans un échantillon biologique, de l'ensemble des trois miARN miR-1-3p, miR-26a-1-3p et miR-487b-3p,
dans laquelle lesdits miARN sont sous-exprimés dans un échantillon biologique d'un patient atteint d'un gliome par rapport à leur expression dans un échantillon biologique d'un sujet sain et lesdits miARN sont surexprimés dans un échantillon biologique d'un patient atteint d'un gliome muté IDH1 par rapport à leur expression dans un échantillon biologique d'un patient atteint d'un gliome de type sauvage IDH1.

2. Méthode selon la revendication 1, dans laquelle l'échantillon biologique est une biopsie liquide.

3. Méthode selon la revendication 2, dans laquelle ladite biopsie liquide est choisie dans le groupe constitué d'un échantillon de sang, d'un échantillon de sérum, d'un échantillon de plasma, d'un échantillon de salive, d'un échantillon d'urine, d'un échantillon de liquide céphalo-rachidien.

4. Méthode selon l'une des revendications 1 à 3, dans laquelle ladite méthode est réalisée en utilisant une ou plusieurs séquences synthétiques complémentaires d'au moins une partie de miR-1-3p, une ou plusieurs séquences synthétiques complémentaires d'au moins une partie de miR-26a-1-3p et une ou plusieurs séquences synthétiques complémentaires d'au moins une partie de miR-487b-3p.

5. Méthode selon la revendication 4, dans laquelle une ou plusieurs séquences synthétiques sont des amorces et/ou des sondes.

6. Méthode selon l'une des revendications 1 à 5, dans laquelle ladite méthode est réalisée par séquençage de nouvelle génération de petits ARN, méthodes basées sur le nanostring, microarray, PCR en temps réel, PCR numérique ou en gouttelettes, méthodes d'hybridation de l'ARN telles que Northern Blot ou Dot Blot.

7. Utilisation d'une combinaison de miARN comme biomarqueur pour le diagnostic *in vitro* de gliomes et/ou pour la discrimination *in vitro* d'un gliome avec une mutation dans le gène IDH1 d'un gliome avec un gène IDH1 de type sauvage, dans lequel ladite combinaison de miARN comprend ou consiste en tous les miR-1-3p, miR-26a-1-3p et miR-487b-3p, dans lequel lesdits miARN sont sous- exprimés dans un échantillon biologique d'un patient atteint d'un gliome par rapport à leur expression dans un échantillon biologique d'un sujet sain et lesdits miARN sont surexprimés dans un échantillon biologique d'un patient atteint d'un gliome muté IDH1 par rapport à leur expression dans un échantillon biologique d'un patient atteint d'un gliome de type sauvage IDH1.

8. Kit pour le diagnostic *in vitro* des gliomes et/ou pour la discrimination *in vitro* d'un gliome avec une mutation dans le gène IDH1 d'un gliome avec un gène IDH1 de type sauvage, ledit kit comprenant ou consistant en trois paires d'amorces et/ou trois sondes pour la détection des trois miARN miR-1-3p, miR-26a-1-3p et miR-487b-3p, à condition que le kit ne comprenne aucune autre paire d'amorces et/ou sonde pour la détection d'autres miARN naturels.
